(19) Europäisches Patentamt
European Patent Office
Office européen des brevets

(11) **EP 4 176 811 A1**

(12) **EUROPEAN PATENT APPLICATION**

(43) Date of publication:
**10.05.2023 Bulletin 2023/19**

(21) Application number: **21206863.9**

(22) Date of filing: **08.11.2021**

(51) International Patent Classification (IPC):
**A61B 5/305** $^{(2021.01)}$ **A61B 5/00** $^{(2006.01)}$

(52) Cooperative Patent Classification (CPC):
**A61B 5/305; A61B 5/7203**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **FRANCK, Christoph Florian**
**Eindhoven (NL)**

(74) Representative: **Philips Intellectual Property & Standards**
**High Tech Campus 52**
**5656 AG Eindhoven (NL)**

(54) **SIGNAL LINEARIZATION IN A MEASUREMENT DEVICE**

(57)    A method for pre-processing electrical sensing signals for compensating for common mode to differential mode conversion caused by signal transfer function nonlinearities. Linearization functions are applied to the input signals to counter the nonlinearities. The method includes a calibration process in which a plurality of sets of standardized test signals are applied to the input channels of the measurement operation, and corresponding test outputs measured for each test signal set. The parameters of the linearization functions are set based on the obtained dataset of test outputs.

FIG. 4

**Description**

FIELD OF THE INVENTION

This invention relates to a method for linearizing input signals to a measurement device for computing a biosignal measurement.

BACKGROUND OF THE INVENTION

A differential measurement is a type of measurement of a physical property (e.g. pressure, temperature, voltage) for which the relevant output from the measurement is a difference, $y$, of the property measured at two points $x_1$, $x_2$, and where the absolute value at each point is not relevant. The value of the physical property at each point may be modelled as comprising a common-mode component, $c$, and a differential-mode component, $d$. The differential mode component, $d$, occurs with opposite signs at each point $x$. For example, the value of a property, x, at two points, and the ideal output measurement, y, from these points might, by way of illustration, be as follows:

$$x_1 = 0.5d + c$$

$$x_2 = -0.5d + c$$

$$y_{ideal} = x_1 - x_2 = 0.5d + c - (-0.5d + c) = d$$

In the ideal case, calculating the difference between $x_1$ and $x_2$ eliminates the common-mode component, $c$, and leaves only the differential-mode component, $d$.
However, in any real system, the signals $x_n$ are subject also to transfer functions $f_n$ before the measurement computation takes place. The transfer functions arise due to behavior of electrical components in the circuit, for example semiconductor components such as diodes, thyristors, transistors, and also more complex circuits such as operational amplifiers and analog-to-digital converters.

$$y_{real} = f_1(x_1) - f_2(x_2) = g(d, c)$$

The transfer functions, $f(x)$, cause the actual output value, $y$, to be a function, $g$, of $d$ as well as of $c$. This is referred to as common-mode to differential-mode conversion (CM2DM), and can significantly degrade the accuracy and reliability of the measurement, since $c$ is usually large in relation to $d$ and even a small fraction of $c$ appearing in the output can dominate the signal, masking part or all of $d$.
If the transfer functions, $f$, are assumed to be linear, CM2DM can be reduced by making them more similar (regardless of the values of the $x_n$). However, if the transfer functions are nonlinear, then making them more similar alone is not sufficient as the input signals may have different offsets and sit at different locations of the nonlinearity, which causes the input signals to experience slightly different small signal gains.

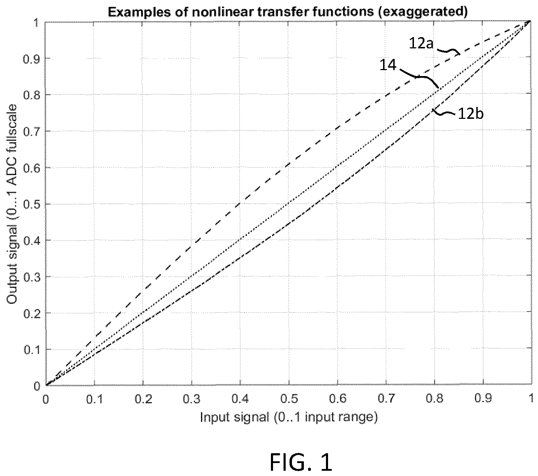

Fig. 1 schematically illustrates example linear 14 and non-linear 12a, 12b transfer functions. As can be seen, for the non-linear transfer function, the resulting gain to the signal is a non-linear function of value of the input signal (x-axis). The y-axis of the depicted graph shows the output value corresponding to a given input signal value (x-axis). The gain, as least for small signals with a constant offset, would correspond to the slope of the curve. Thus, the gain applied by the transfer function will depend upon the offset of the input signal, since this offset will change the location of the baseline of the input signal along the non-linear transfer function.
The offset may be understood as a part of the differential component, $d$. The main cause of the offset is the behavior of the skin-electrode interface, which may act as an inadvertent battery, generating electric potentials of up to several hundred millivolts. The user of the measurement device is usually not interested in this offset, but in the components of $d$ that are due to physiological causes. The physiological electrical activity is most typically in the form of AC signal components. The offsets may in some cases be "true" differential components, but this is not relevant if the user is only interested in the physiological (AC) components of the signal.
Nonlinearity of the input channel transfer functions can be caused by components in the input path that may exhibit significant nonlinear behavior, ranging from passive semiconductors (e.g. diodes or thyristors used for overvoltage protection), operational amplifiers and analog-to-digital converters (ADCs).

The nonlinear behavior of the input channels can lead to significant CM2DM even if the linear components of the transfer functions are equalized.

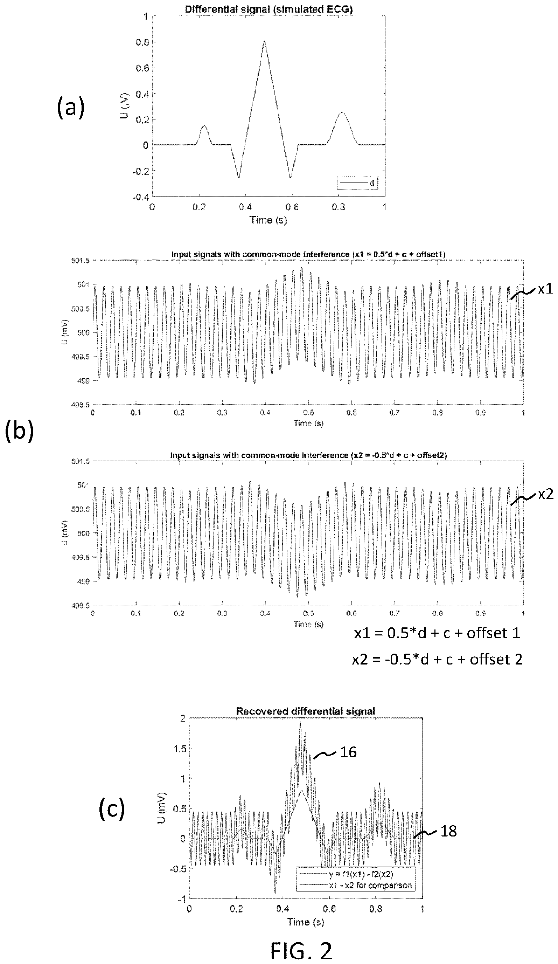

By way of illustration, Fig. 2 shows a representation of how non-linear transfer functions may affect the computed differential signal in a measurement system. Fig. 2(a) shows the true differential signal, $d$ (simulated). Fig. 2(b) shows the two sensor signals, $x1$ and $x2$, including for each signal the common mode, $c$, the differential mode, $d$, and the respective offset.

Fig. 2(c) shows the resulting recovered differential signal 16 computed by the measurement device, wherein each input signal has been the subject of a transfer function (f1 and f2 respectively), and the differential signal has been computed simply as the difference of the two input signals. Fig. 2(c) also shows the true differential signal 18 on the same axes for ease of comparison. As can be seen from Fig. 2(c), the computed differential signal comprises a large amount of noise of an amplitude which almost exceeds the magnitude of the differential signal itself, resulting in a signal-to-noise ratio which is poor.

Differential measurements are commonly utilized within electromedical applications. By way of example, well-known measurement modalities such as electrocardiography and electroencephalography (electrical activity of the heart and the brain, respectively) are differential measurements. Other, less well-known measurement modalities which are also differential measurements include electroneurography and electromyography. Emerging electroanatomical imaging applications are also, in part, based on differential voltage measurements.

The signal of interest in electromedical applications commonly is a time-varying (AC) signal generated by physiological activity of muscle or nerve tissue.

The source signal comprises a common mode component, $c$, a differential mode component, $d$, and an offset, $k$, and the sampled signal additionally has been the subject of a transfer function. The offset, $k$, can differ for each source signal. The offset can be caused for example by electrochemical properties of the interface between the patient and the measurement system. For example, for an ECG measurement apparatus, this would typically consist of adhesive electrodes applied to the patient's skin. The most prevalent source of the common-mode components is power line interference which can couple into the measurement by different possible paths/routes. In particular, interference can couple into the measurement by way of the patient (capacitively if the patient is in the proximity of the interference source, or directly by conduction if the patient is in contact with an electrical ground), via the cabling (more so if the cables are not fully shielded), or the measurement electronics (the patient module is usually galvanically isolated, but still has a capacitive connection to ground).

Other sources of common-mode interference may include for example electrical devices operated in the vicinity or in contact with the patient, the measurement device, or merely on the same power circuit, whose properties (frequency, amplitude, variation over time, etc.) may differ significantly from typical power line interference patterns.

Common mode interference is addressed in state of the art devices, by means of a processing operation in which a portion of the common mode component is removed (rejected) to compensate for estimated interference contribution. The proportion of the common mode component which is rejected is known as the common-mode rejection ratio.

For example, known devices may address common mode interference with measures like driven-right-leg loops, shielding of recorders and cabling, use of precision components, as well as linear equalization of the input channels.

In case interference does occur in a clinical setting, sometimes a device may include functionality to issue instructions to an operator to perform skin preparation before attaching the electrodes, to verify that the electrodes are attached properly to the patient, to replace the electrodes regularly and to check for and remove electrical devices in the vicinity of the patient that could be possible sources of interference. If this fails to reduce the interference, further suggestions might be issued such as to rotate the patient or the equipment by 90 degrees.

Nonlinearity is only considered to affect the gain of the output, and production test limits are set to keep the output gain inside the allowed range. This ensures the device is compliant with applicable standards and regulations, but still allows significant CM2DM to occur due to transfer function nonlinearity.

State of the art electrophysiological recorders do not take into account the effects of possible nonlinear input channel transfer functions on the common-mode rejection ratio of the device. A method for addressing this non-linearity would be of value.

## SUMMARY OF THE INVENTION

The invention is defined by the claims.

According to an aspect of the invention, there is provided a computer-implemented method for implementation on a measurement device. The method comprises a differential measurement operation, comprising:

> reading in signals from at least two input channels of the measurement device, the signal from each channel being received from a respective sensor element;
> applying a linearization function to each of the input channels, the linearization function having adjustable parameters;

applying a computation algorithm to the input channels subsequent to applying the linearization function, to derive at least one output measurement;

outputting a data signal indicative of the output measurement.

The method further comprises a calibration operation, performed at a different time than the measurement operation, the calibration operation comprising:

applying a plurality of sets of constant-value calibration input signals to the input channels of the measurement device;

applying the computation algorithm to each set of the calibration input signals without applying the linearization function, and recording each respective output measurement, to form a calibration dataset; and

fitting the adjustable parameters of the linearization function based on the calibration dataset.

Thus, the method is based on applying linearization to the input signal channels, and where the parameters of the linearization function are configured based on a calibration operation in which standardized test signals are applied to the input channels of the measurement device, and the corresponding output measurement signals are recorded. Based on a relationship between the test inputs and the measured test outputs, an appropriate set of linearization function parameters can be determined.

The signal may be an electrophysiological signal.

In some embodiments, the linearization function may be a polynomial function.

In some embodiments, the signal received at each input channel is assumed to represent a true sensing signal which has been processed by a non-linear transfer function, and wherein fitting the adjustable parameters of the linearization function comprises an optimization process in which an error between a true output measurement for a set of calibration input signals, as would be obtained if the non-linear transfer function were not applied to the input signals, and the actual output measurement as recorded in the calibration dataset, is minimized.

It is noted that the non-linearity of the transfer function typically arises predominantly due to effects of electrical components inside the measurement device itself, rather than within the sensing apparatus outside of the measurement device. Asymmetries introduced by the external part of the measurement setup are predominantly linear effects, since the cables can be modelled as a network of linear components such as resistors and capacitors. Thus, it is for this reason that that the calibration process can be achieved through the application of the constant value calibration reference signals directly into the input ports: the non-linearities are induced within the electronics of the measurement device itself (e.g. from protection diodes/thyristors, operational amplifiers, analog-to-digital converters, power supplies).

In some embodiments, the computation algorithm may comprise application of one or more vectors to the input channels, each vector defining a mapping from the set of input channels to an output measurement. The output measurement may be a linear combination of the input channels, with weightings of the linear combination defined by elements of the vector. The calibration of the linearization function parameters may be performed based in part on the one or more vectors. In other words, the linearization function calibration is performed based in part on the computation algorithm which is employed in computing the output measurements. For example, in simple terms, the calibration of the linearization function parameters may comprise a process of minimizing a disparity or error between the expected output(s) from application of the vectors to the calibration input signals and the actual output(s) from application of the vectors to the calibration input signals.

In some embodiments, the measurement operation may comprise application of a plurality of said output vectors for deriving a plurality of different output measurements.

In some embodiments, the measurement device may comprise more than two input channels. In some embodiments, each of the plurality of output vectors defines a mapping from only a subset of the input channels to a respective output measurement.

In some embodiments, the measurement operation comprises reference to a dataset of multiple different linearization functions, where each linearization function is associated with only a subset of the plurality of output vectors, and wherein only the linearization function associated with a given output vector is applied to a given input channel in advance of applying the respective output vector. In other words, the different input channels may be subject to different linearization functions, and each input channel may be processed with more than one linearization function (in separate, parallel operations), in dependence upon the particular output vector which is to be applied.

For example, a simple illustrative case might be a system with four input channels, and wherein the computation algorithm comprises application of two vectors, and wherein the first vector computes a difference between the first and second input channels and the second vector computes the different between the third and fourth vector.

In this case, optimal linearization of the first and second input channels to which the first vector is applied might be performed separately from linearization of the third and fourth input channels to which the second vector is applied, since, for example, the calibration procedure does not need to consider how well the linearity matches for the first and fourth input channel, as this pair will never be used together in calculating one of the two vectors.

In some embodiments, the input channels are each assumed to comprise a differential mode component, a common-mode component, and an offset, and wherein the calibration procedure comprises making reference to a predetermined probability distribution for the offsets of the input channels. This probability distribution is used either explicitly (actively) or implicitly in the calibration algorithms, and guides selection or determination of an appropriate estimated offset for use in the linearization function(s).

For example, the calibration procedure first records the calibration dataset obtained by applying the computation algorithm to each set of the calibration input signals without applying the linearization function, the calibration dataset comprising a respective calibration output value for each applied calibration input signal, and for each of a range of possible offset values. The calibration procedure may then comprise generating a cost function, which could, by way of illustration, be computed as the sum of the absolute differences of the slopes of the calibration output values as a function of the calibration input values for all of the combinations of possible offsets. In other words, for each possible combination of offsets of the input channels, a respective set of calibration output values as a function of applied calibration input signals is obtained. Each of these sets of calibration output values defines a slope with respect to (as a function of) the applied calibration input values. The cost function may be computed based on the differences of these slopes for the different combinations of offsets, with the optimisation (the fitting procedure) based on minimising this cost function (to thereby linearize the input channels).

If all the combinations of possible offsets are equally likely when the device is in normal operation, then no further refinement of the cost function is possible. The optimal fitting of the linearization function is that which results in the slopes of the input channel transfer functions (i.e. the calibration output values as a function of the applied calibration input signals) being identical for all combinations of input channel offsets. This is equivalent to just linearizing each individual input channel.

If there is prior knowledge about some combinations of offsets being more (or less) likely than others, for example due to the presence of an additional circuit such as a Driven Right Leg (DRL) circuit, then this knowledge can be applied to give more weight to likely offset combinations, and less weight to unlikely ones. Depending on the type of nonlinearity and the linearization function, this may reduce CM2DM for the expected operating conditions, at the cost of worse CM2DM in unlikely or abnormal operating conditions.

In some embodiments, the linearization function, and the calibration operation, is configured such that the calibrated linearization function provides more optimal linearization for the set of offsets which has highest probability in the probability distribution, and least optimal linearization for the set of offsets which has lowest probability in the probability distribution.

For example, the signal received at each input channel may be assumed to represent a true sensing signal which has been processed by a non-linear transfer function, and wherein fitting the adjustable parameters of the linearization function comprises an optimization process in which an error between a true output measurement for a set of calibration input signals, as would be obtained if the non-linear transfer function were not applied to the input signals, and the actual output measurement as recorded in the calibration dataset, is minimized. In this case, more optimal linearization for a set of possible offsets means a smaller average error in the output measurement after calibration for input signals having said possible set of offsets, and a less optimal linearization for a set of possible offsets means a greater average error in the output measurement after calibration for input signals having said possible set of offsets.

This allows for all possible scenarios to be accounted for, but provides a smooth degradation in the quality of linearization as the offsets change from more common to less common sets.

A further aspect of the invention provides a computer program product comprising code means configured, when run on a processor, to cause the processor to perform a method in accordance with any example or embodiment outlined above or described further below, or in accordance with any claim of this application. The code may cause the processor to perform the measurement operation of the method when the processor is operatively coupled with two or more sensor elements, acting as sources of the input signal channels. The code may cause the processor to perform the calibration operation of the method when the processor is successively ported/supplied a series of sets of calibration input signals.

A further aspect of the invention provides a processing arrangement, comprising: an input/output; and one or more processors. The one or more processors may be operable in at least a first mode and a second mode. In the first mode, the one or more processors may be adapted to perform a differential measurement operation, comprising at least the following steps:

reading in signals from at least two input channels of the measurement device, the signal from each channel being received from a respective sensor element;
applying a linearization function to each of the input channels, the linearization function having adjustable parameters;
applying a computation algorithm to the input channels subsequent to applying the linearization function, to derive at least one output measurement;
outputting a data signal indicative of the output measurement;

In the second mode, the one or more processors may be configured to perform a calibration operation, comprising at least the following steps:

applying a plurality of sets of constant-value calibration input signals to the input channels of the measurement device;
applying the computation algorithm to each set of the calibration input signals without applying the linearization function, and recording each respective output measurement, to form a calibration dataset; and
fitting the adjustable parameters of the linearization function based on the calibration dataset.

A further aspect of the invention provides a measurement device comprising:
at least two signal input ports for simultaneously reading in at least two input signal channels from respective sensor elements; and a processing arrangement as described above, or in accordance with any embodiment or example outlined in this disclosure, or in accordance with any claim of this application.

A further aspect of the invention provides a system comprising: a measurement device as described above, or in accordance with any embodiment or example outlined in this disclosure, or in accordance with any claim of this application. The system further comprises a signal measurement apparatus comprising at least two sensor elements for connection to the signal input ports.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates example linear and non-linear transfer functions;
Fig. 2 illustrates differential and common mode interference;
Fig. 3 shows an example system and processing arrangement in accordance with one or more embodiments of the invention;
Fig. 4 schematically illustrates an example processing workflow of a measurement operation performed by the processing arrangement in accordance with one or more embodiments of the invention;
Fig. 5 schematically illustrates an example processing workflow of a calibration operation performed by the processing arrangement in accordance with one or more embodiments of the invention;
Fig. 6 illustrates an example probability distribution for offsets of a set of two input signal channels;
Fig. 7 illustrates in more detail examples of output signals obtained in low, medium and high probability offset conditions, when a probability density of offsets is not taken into account in linearization;
Fig. 8 illustrates in more detail examples of output signals obtained in low, medium and high probability offset conditions, when a probability density of offsets is taken into account in linearization;
Fig. 9 illustrates an example processing flow according to one example, in which a single linearization function is applied to each input signal channel and a single output vector is applied to both input channels; and
Fig. 10 illustrates a further example processing flow according to a further example, in which two output vectors are applied to different selected combinations of a set of three input channels.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.
The invention provides a method for pre-processing electrical sensing signals for compensating for common mode to differential mode conversion caused by signal transfer function nonlinearities. Linearization functions are applied to the input signals to counter the nonlinearities. The method includes a calibration process in which a plurality of sets of standardized test signals are applied to the input channels of the measurement operation, and corresponding test outputs measured for each test signal set. The parameters of the linearization functions are set based on the obtained dataset of test outputs.

By way of further illustration of the concept, according one example set of preferred (but non-exhaustive) embodiments, details of which will be further elaborated in passages to follow, there is provided a method which seeks to solve the problem of common-mode to differential-mode conversion by input channel transfer function nonlinearities by providing a measurement device that applies one or more linearization functions $h_n(x)$ to the input signals before calculating the difference or vector that defines the output measurement. The linearization functions use parameters which are determined in a calibration procedure by applying a sequence of constant input signals to the inputs of the device and recording the (raw/ non-linearized) output. The dataset of calibration input and output values, along with the information about the vectors used by the device in computing the measurements, and preferably a predetermined statistical distribution of expected signal offset values (or combinations of offset values) assumed to be comprised as components in each input signal, is then used to calculate linearization parameters that provide optimal linearization for likely combinations of channel offset values, and less optimal linearization for uncommon combinations.

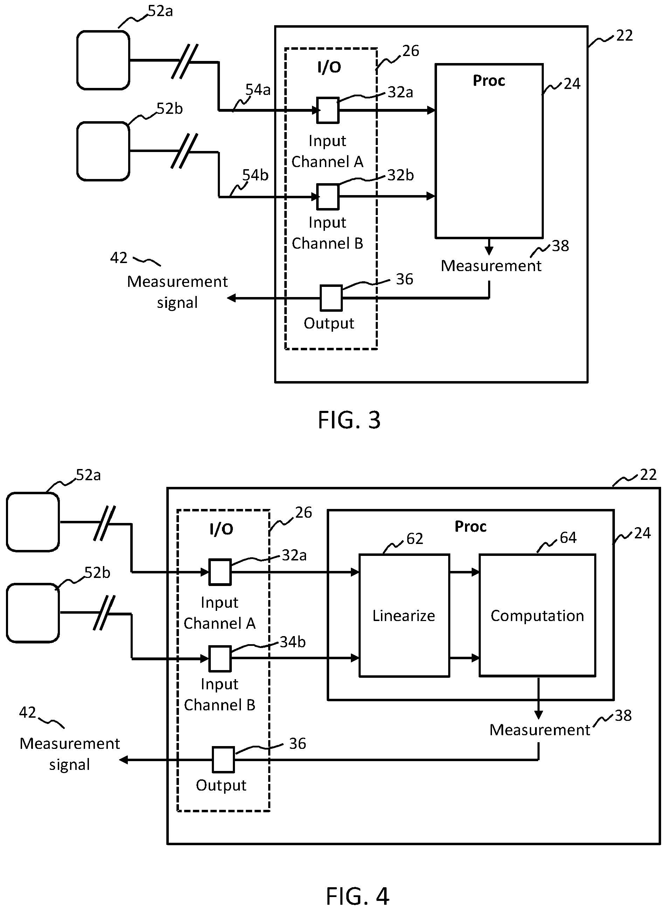

Fig. 3 shows an example processing arrangement 22 and system in accordance with one or more embodiments of the present invention.

The processing arrangement 22 comprises an input/output (I/O) 26 and one or more processors 24. The I/O has in this example two input channels 32a, 32b, and one output channel 36. However, the I/O may have more than two input channels in other examples, and may have more than two output channels.

A respective sensor element 52a, 52b is connected to each input channel 32a, 32b. Each sensor element is adapted for acquiring a biosignal at a location on a subject's body. By way of example, the sensor elements may each be ECG electrodes. The signal from each sensor element 52a, 52b provides a respective input signal 54a, 54b to the input channels 32a, 32b.

The one or more processors 24 (just one is shown in Fig. 3, but an assembly of multiple processors might in practice be used) receive the input signals from the input channels 32a, 32b and perform processing of the signals to derive one or more output measurements 38, and these are then exported via the output channel 36 as a measurement data signal 42.

The one or more processors 24 are in particular adapted to perform a computer-implemented method which includes a differential measurement operation and a calibration operation, performed at different respective times.

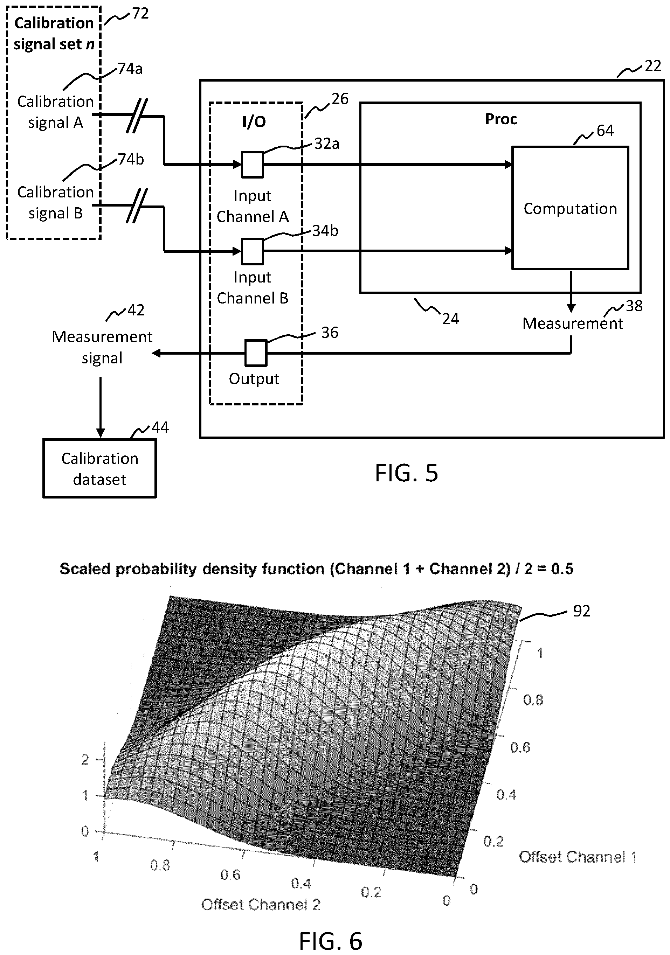

Fig. 4 schematically illustrates the processing workflow for the differential measurement operation. Fig. 5 schematically illustrates the processing workflow for the calibration operation.

The differential measurement operation comprises the following steps, performed by the one or more processors 24.

The measurement operation comprises reading in signals from the at least two input channels (Channel A 32a, and Channel B 32b) of the measurement device. The signal from each channel is received from a respective sensor element 52a, 52b. The measurement device may include an analog-to-digital conversion means which is applied to the received input signals to digitize the input signals.

The measurement operation further comprises applying a linearization function 62 to each of the input channels, the linearization function having adjustable parameters.

The measurement operation further comprises applying a computation algorithm 64 to the input channels subsequent to applying the linearization function 62, to derive at least one output measurement 38. The input channels fed to the computation algorithm are preferably single-ended input channels, and the output from the measurement algorithm is preferably a single ended output signal.

A data signal 42 is output indicative of the output measurement 38. This data signal is output via an output channel or port 36 of the input/output 26.

The measurement operation may be performed fully or partly using hardware (ASIC, FPGA) and/or software running on a microprocessor (microcontroller, digital signal processor).

The one or more processors are further operable to perform a calibration operation, which comprises the following steps, illustrated in Fig. 5.

The calibration operation comprises applying a plurality, n, sets 72 of constant-value calibration input signals 74a, 74b to the input channels 32a. 32b of the measurement device 22. The $n$ sets are applied one-by-one, in a sequence. The calibration input signals of each respective set are applied simultaneously to the input channels. The constant signal values of the calibration input signals may be otherwise referred to as the 'calibration points'.

The calibration operation further comprises applying the computation algorithm 64 to each set 72 of the calibration input signals 74a, 74b without applying the linearization function, and recording each respective output measurement 38, to form a calibration dataset 44. Each respective output measurement 38 may be output in the form of a data signal 42 via the output channel 36 of the I/O 26. The calibration dataset may be stored on a local datastore of the device 22, or a remote datastore.

The calibration operation further comprises fitting the adjustable parameters of the linearization function 62 based on the calibration dataset 44.

With regards to the measurement operation and calibration operation, the processing arrangement 22 may be operable in at least a first and second mode, wherein, in the first mode, the processing arrangement is adapted to perform the measurement operation, and in the second mode, the processing arrangement is adapted to perform the calibration operation.

The calibration operation is performed at a different time to the measurement operation. The calibration operation may be performed only once, before use for measurement, e.g. in the factory, or during a pre-operation initialization. The calibration operation alternatively may be performed with the measurement device in situ. It may be performed recurrently.

The measurement device 22 may include a user interface which permits a user to provide a user input to control selection of the first or second mode. These mode selection options may only be available in a certain primary configuration or mode of the device, e.g. when it is put first into a Config or Setup mode. In some examples, the measurement device may be adapted to enter the calibration mode responsive to receiving a pre-determined control signal from a separate secondary device via a data connection. The data connection may be facilitated through a dedicated Control or Config port comprised by the measurement device. The data connection may be removable from the measurement device port. For example, a user might be supplied with a separate device which is connectable to the measurement device to stimulate execution of the calibration steps in an automated manner. In some examples, the calibration operation may be an algorithm or computer program which is stored on the secondary device, in which case the provided invention may comprise the measurement device in combination with the secondary device.

The ability to execute the calibration operation in situ, after manufacture, allows for recalibration to be regularly performed, to account for component aging and other slow changes of the transfer function nonlinearity over time.

In further examples, the two modes may not freely selectable by an end-user. For example, the device may be placed in the measurement operation mode when shipped, and wherein the calibration mode is accessible only via a specialized data input port, and/or with a key, which can be utilized in the factory.

The measurement device in some examples may be an ECG measurement device. The measurement device in some examples may comprise a user interface for use in displaying the measurements 28, and/or for receiving user control commands.

With regards to the computation algorithm 64 applied in the measurement operation, this may comprise application of one or more vectors to the input channels, each vector defining a mapping from the set of input channels to an output measurement, where the output measurement is a linear combination of the input channels with weightings defined by elements of the vector. The one or more vectors which are applied in the measurement operation may be stored in a local datastore, e.g. stored in advance during manufacture or setup, and retrieved during execution of the measurement operation.

The vectors may define linear combinations of the input channels, where the sum of the weighting coefficients is zero. For example, in the simplest case, the output measurement may be simply a difference of the two input signals. In this case, the coefficients are -1 and 1, which sum to zero.

In some embodiments the measurement operation may comprise application of a plurality of said output vectors for deriving a plurality of different output measurements 38.

In some embodiments, the measurement device comprises more than two input channels, wherein each of the plurality of output vectors defines a mapping from only a subset of the input channels.

The minimum list of features as described above, when combined, result in a measurement device that is more resistant to interference and provides undisturbed, clean output signals in circumstances where a device not using the method of the invention would show an output signal degraded by significant interference. This leads to more accurate measurements, and therefore more accurate diagnosis and consequent therapy.

Further details relating to the linearization will now be described.

According to one simple example, the one or more linearization functions $h_n(x)$ may be polynomial functions, and wherein the adjustable parameters of the linearization functions are the coefficients of the polynomial, e.g.

$$h(x) = ax^3 + bx^2 + cx + d$$

This is computationally efficient since only a small number of multiplication operations are necessary to calculate the value of a polynomial. Lower or higher order polynomials may be used, with the selection based on a preferred balance between computational efficiency (lower order), and precision (higher order).

The one or more linearization functions are preferably monolithic functions, but may instead be piecewise functions, for example cubic or higher-order splines, as these should exhibit sufficient smoothness to work efficiently.

In further embodiments, the one or more linearization functions may use other functions as the basis for $h_n(x)$, for example trigonometric functions, for example the hyperbolic tangent tanh(x), e.g.

$$h(x) = a * \tanh(b * x + c) + d$$

Calculation of the coefficients, based on the compiled calibration dataset 44, may be cast as a convex optimization problem. This can be readily solved with automated function fitting algorithms and solvers, such as regression algorithms.

One implementation, in accordance with at least one set of embodiments, will now be outlined in more detail. In this set of embodiments, the computation of the linearization parameters utilizes the one or more vectors used in computing the measurements in the measurement operation.

In simple terms, during the calibration operation, the following information is known: the value of each pair of calibration input signals, the computation being performed on them, and the measurement result which is provided as an output. Based on the knowledge of the computation being performed on them (i.e. the vectors being applied to the input signals), it is theoretically possible to identify what would be an "expected" output measurement in the absence of CM2DM interference. Thus the disparity between expected and measured output can be used to guide the fitting of the linearization function. The fitting of the linearization function in other words is performed with an aim of minimizing an error between a "true" expected measurement output, based on the known inputs and the known computation function 64, and the actual measurement output during the calibration. Since multiple sets of calibration signals are applied, ideally defining regularly incrementing or decrementing differentials, the non-linearity of the error is exhibited in the calibration dataset, so that it is taken account of properly in the fitting of the linearization function.

Thus, in summary, the signal received at each input channel is assumed to represent a true sensing signal which has been processed by a non-linear transfer function, and wherein fitting the adjustable parameters of the linearization function comprises minimizing, for the calibration dataset, an error between a theoretical true output measurement for each calibration input signal, as would be obtained if the non-linear transfer function were not applied to the input signals, and the actual output measurement as recorded in the calibration dataset.

With regards to the calibration input signals, by way of further illustration, for the linearization of a single input channel, the set of calibration points (i.e. the signal values of the calibration input signals) is just the set of calibration input signals for the single channel. For example, if the input range is 0V to 1.0V, the calibration signal values may be selected as 0V, 0.25V, 0.5V, 0.75V, and 1.0V. This list of voltages corresponds to the set of calibration points. This also holds in multi-channel cases where each input channel is linearized independently of the others.

In the case of a multi-channel linearization in which combinations of input channels are considered, the set of calibration points consists of combinations of calibration input signal values. In a case with two channels and the above list of voltages, the full set of calibration points consists of all $5^2=25$ combinations (also called the Cartesian product) of {0V, 0.25V, 0.5V, 0.75V, 1.0V} $\times$ {0V, 0.25V, 0.5V, 0.75V, 1.0V}, i.e. (0V, 0V), (0V, 0.25V), ..., (0.25V, 0V), (0.25V, 0.25V), ... (1.0V, 1.0V). The size of the set rises exponentially with the number of input channels - in a system with 10 input channels (e.g. a 12-lead ECG recorder) and five test voltages, the full set of calibration points contains almost ten million elements.

In a simplest case, all of these combinations are taken into account in the process of fitting the linearization function(s). However, while state of the art processors are able to handle such volumes of data processing, the calibration operation can be accelerated if only a subset of the possible calibration points are used in the fitting of the linearization function(s). Elements can be excluded from the set of data points based on knowledge of the vectors used in the measurement operation (e.g. knowledge that an ECG measurement operation does not form vectors containing more than one input chest lead).

Elements can also be excluded based on knowledge that the elements do not represent conditions where the device is expected to produce valid output, or do not occur in practice, e.g. due to the presence of a Driven Right Leg (DRL) circuit which forces the mean of the input channels to 0.5 V, which means that combinations of input signals such as (1.0V, 1.0V) and (0V, 0V) can be disregarded in the calculations. For example, during normal operation, the combinations (0V, 1.0V), (0.25V, 0.75V), (0.5V, 0.5V), (0.75V, 0.25V), and (1.0V, 0V) are most relevant, as any other combination represents a transitory or abnormal/faulty state.

One difficulty, as noted above, is that in actual use, the sensor measurement input signals 54a, 54b can be expected to each have a certain offset, in addition to the standard differential mode and common mode components. The offsets depend upon the physical properties of the apparatus setup, as described above. The offsets cause significant difficulty for a processing operation involving application of a non-linear function (i.e. the linearization function, *h(x)*), since the offset will cause a corresponding non-linear distortion in the output measurement signal, if it is not taken into account in the linearization function itself. The offsets however cannot be known in advance. Therefore, the most optimal solution is to utilize an estimated probability distribution of input channel offsets, and configure or tune the parameters of the linearization function so that at least offsets within a highest probability region of the distribution do not cause very significant distortion in the output measurement signal. Ideally, the linearization function is also tuned so that, as a function of the offset of the input signals, the resulting offset-related distortion changes in a relatively smooth manner.

In other words, it is preferred that, as the (possible) offset of a signal moves from the more probable range to the less probable range, there is not a large jump or discontinuity in the output measurement signal, but rather the resulting signal distortion increases smoothly, and the signal accuracy therefore degrades smoothly.

Thus, according to the aforementioned set of embodiments, the input channels are each assumed to comprise a differential mode component, a common-mode component, and an offset, and wherein the calibration procedure comprises reference to a predetermined probability distribution for the offsets of the input channels.

In other words, the calibration dataset 44 of calibration input and output values, along with the information about the vectors used by the device in computing the measurements, and a predetermined statistical distribution of input signal offset values (or combinations of offset values) assumed to be comprised as components in each input signal, is then used to calculate linearization parameters that provide optimal linearization for likely combinations of channel offset values, and less optimal linearization for uncommon combinations.

In particular, in some embodiments, the linearization function, and the calibration operation, is configured such that the calibrated linearization function provides more optimal linearization for a set of offsets which has a highest probability in the probability distribution, and less optimal linearization for a set of offsets which has lower probability in the probability distribution.

In this context, more optimal linearization for a set of possible offsets means, after calibration, a smaller average error in the output measurement for input signals having said possible set of offsets, and a less optimal linearization for a set of possible offsets means, after calibration, a greater average error in the output measurement for input signals having said possible set of offsets.

In an application with only a single channel, the quality of the linearization would be measured by the deviation of the transfer function (that is, the function of the calibration output values as a function of the applied calibration input signals) from an ideal linear function.

However, in the type of application described in the invention disclosure, while simply linearizing each input channel this way may provide a significant improvement over the uncalibrated state, it may not be the most optimal result with respect to reducing common-mode to differential-mode conversion with the given degrees of freedom of the linearization function.

In particular, an optimal linearization is achieved where there is an absence of CM2DM during expected operating conditions. Expected operating conditions can be influenced by factors such as the presence in the input signal path of a Driven-Right-Leg (DRL) feedback loop (see below), which drives the sum of the input signal potentials to a fixed value. The optimization algorithm could for example assign a larger weight to reducing CM2DM in conditions where the sum of the input electrode potentials is close to this fixed value, and give less or no weight to conditions where the sum is different from the expected value.

By way of illustration, Fig. 6 shows an example probability distribution for offsets of a set of input channels which comprises only a first (Channel A) and second (Channel B) input channel, and where the system setup includes a driven right leg (DRL) circuit The graph shows a scaled probability density function for a system in which (Channel A + Channel B) / 2 = 0.5.

To explain further, electrophysiological measurement devices often incorporate a feedback circuit which feeds a negative sum of the input electrode potentials (plus or minus a constant offset) back to the patient over a designated electrode. In ECGs, this is often the right leg electrode, which is why the circuit is referred to as a "driven right leg/DRL" or "right leg drive/RLD" circuit, although in general an electrode attached to any body part can be used. In other measurements, the terms "active electrode", "reference electrode" or "driven electrode" may be used, and the term "feedback circuit" may be used in place of DRL circuit.

The main purpose of the DRL circuit is to reduce the common mode signal. This generally works very effectively for low frequencies, but its effectiveness diminishes at higher frequencies due to control loop instability and limitations on the current that may flow from the device to the patient.

Another effect of the DRL circuit is to establish an operating point. The DRL circuit will drive the input electrode potentials to a state where their sum is approximately equal to a constant offset. Under normal conditions, this operating point will be reached quickly - within less than one second. Thus, in this scenario, the input channel voltages may be assumed to be close to the calibration points (1.0V, 0V), (0.75V, 0.25V), (0.5V, 0.5V), (0.25, 0.75) and (0V, 1.0V), for a constant offset of 0.5V in this example.

This for example makes use of a polynomial as the linearization function a reasonable choice, because any positive integer power of x is monotonic for x >= 0, where x is the value of the input signal to the input channel.

It is noted however that the ADC connected between the input port and the processor often uses bipolar sampling and the midpoint of the ADC range is 0V. This needs to be considered when choosing and applying the linearization function.

With reference to Fig. 6, the measurement range is from 0 to 1 and the midpoint is 0.5. The right leg drive operates to maintain a mean of the two channels at 0.5. In this particular case, this would imply that input channel offset combinations that sum to zero are more likely, and input channel offset combinations that sum to a nonzero value are unlikely, as the latter only occurs during the settling of the DRL control loop, which is a brief transitory period, or during abnormal operating conditions. Thus, this may in some embodiments be taken into account when determining the probability distribution of offsets. For example, the optimization process can disregard the quality of the linearization for these situations, especially if the linearization can be further improved for the more usual situations.

Additionally, if all electrodes are attached equally well and have been in use for the same length of time, the DC offsets they produce will be approximately equal. On the other hand, if the electrodes have different ages or attachment qualities, their DC offsets are more likely to be different. The optimization process can optimize the linearization more strongly for a situation where x1≈0.5, x2≈0.5 (where x1 and x2 are first and second input channel signals) but it should not completely disregard situations where this does not hold, as this may indicate a poor measurement setup, but not an abnormal operating condition.

The probability distribution function of offsets is determined in advance and stored for later use. It may be determined through empirical testing, or through theoretical/analytical modelling or simulation.

It is not essential that the linearization function fitting take into account a probability distribution of input signal channel offsets. However, if the calculation of the linearization parameters does not consider the probability distribution function, the result may (paradoxically) be that the linearization result may be worse for likely conditions and better for improbable ones.

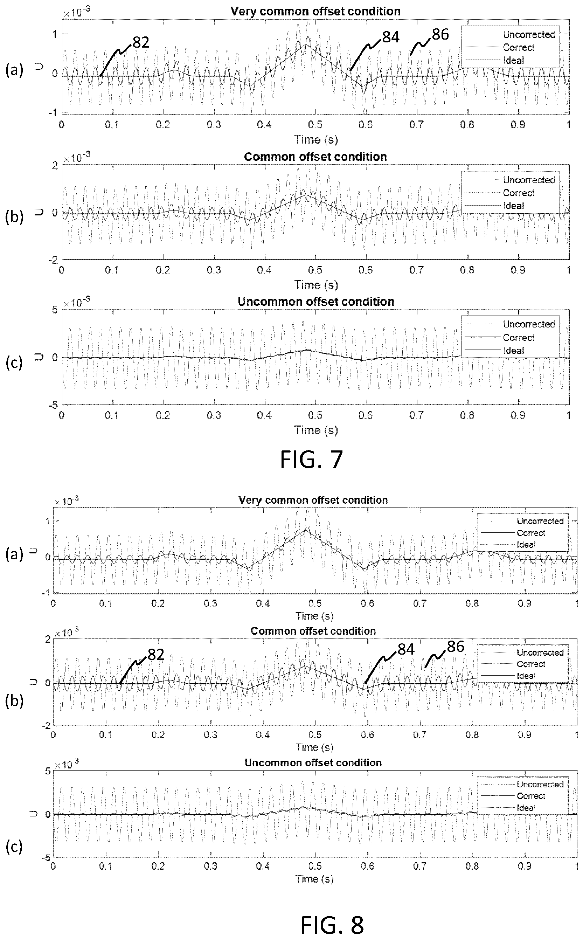

This is illustrated for example in Figs. 7 and 8. Each graph in each figure shows a "true" 82 input signal from a sensor element, an actual recorded (uncorrected) input signal 84, and the corrected input signal 86 after application of the linearization function.

Fig. 7 shows the result for a series of different probability offset conditions, in a case where the linearization function has not been fitted so as to take account of an expected probability distribution of signal offsets. Fig. 8 shows the result for the same series of different probability offset conditions, in a case where the linearization function has been fitted so as to take account of an expected probability distribution of signal offsets. It can be seen that in the scenario of Fig. 7, the common-mode interference suppression is actually significantly better in the more unlikely offset scenario than in the more likely ones. By contrast, in the case of Fig. 8, the correction of the signal is best in the most likely offset scenario and worst in the least likely offset scenario (which is clearly a preferred outcome).

The probability density function can be used in the fitting of the linearization function(s), so as to apply higher weightings to likely operating conditions in the cost function used in the fitting/optimisation, and lower weighting to unlikely or abnormal operating conditions. Operating conditions in this context refers for example to more likely and less likely combinations of input signal offsets and/or more likely or less likely combinations of input signal values.

These applied weightings will cause the optimization process to improve linearization for states with a high weight at the expense of states with lesser weight.

To explain this further, while it has been mentioned above that certain unlikely combinations of possible input channel signals could be excluded entirely from the calibration dataset, to speed up computation of the linearization function fitting, the use of a probability distribution allows a more refined approach. The probability distribution allows different combinations of input signals and/or different combinations of offset values to be assigned different weightings in the optimization operation (i.e. the fitting of the linearization function parameters), according to their estimated probability of occurring in normal operating conditions.

For example, continuing with the two-channel example which was discussed above, input voltages near the (0.5V, 0.5V) calibration point might represent operating conditions with fresh electrodes and proper attachment to the patients skin, while input voltages close to the points (1.0V, 0V), (0V, 1.0V), (0.25V, 0.75V) and (0.75V, 0.25V) may occur in situations when the electrodes have degraded due to prolonged use (the latter generally being less likely than the former). Additionally, input voltages close to the points (0.25V, 0.5V), (0.75V, 0.5V), (0.5V, 0.25V), (0.5V, 0.75V) may represent transitory states during which the device should still produce noise-free output.

In order to reduce CM2DM due to channel nonlinearity, the optimization algorithm used in the calibration operation may seek parameters for the linearization function that make the slopes (or some approximation of the slopes) of the output measurement values as a function of applied sets of input channel signals as similar as possible at each calibration point (where a calibration points means a particular combination of input channel signals).

One way to describe this cost function mathematically is the sum of the absolute differences of these slopes at each calibration point.

For example, the calibration procedure first records the calibration dataset obtained by applying the computation algorithm to each set of the calibration input signals (i.e. at each calibration point) without applying the linearization function, the calibration dataset comprising a respective calibration output value for each applied calibration input signal, and for each of a range of possible offset values. The calibration procedure may then comprise generating a cost function, which could, by way of illustration, be computed as the sum of the absolute differences of the slopes of the calibration output values as a function of the calibration input values for all of the combinations of possible offsets. In other words, for each possible combination of offsets of the input channels, a respective set of calibration output values as a function of applied calibration input signals is obtained. Each of these sets of calibration output values defines a slope with respect to (as a function of) the applied calibration input values. The cost function may be computed based on the differences of these slopes for the different combinations of offsets, with the optimisation (the fitting procedure) based on minimising this cost function (to thereby linearize the input channels).

In order to bias the optimization process towards best CM2DM reduction at the most common operating points, the absolute difference of the slopes at each calibration point can be multiplied by a weighting factor that correlates to the probability of operating near this calibration point. The probability can be expressed as the probability density function (PDF). The probabilities used in the probability distribution function may be estimated.

Each calibration point corresponds to a set of applied calibration input signals, where each calibration input signal include for example a sum of a main signal component and a calibration offset component. In this way, the calibration can take account of different possible (differential mode) input signal values, and different possible combinations of offsets.

With regards to the probability distribution for the offsets, this can be determined in advance of the measurement and calibration operations. Different embodiments may differ in the assumptions they make about the probability density function of the offsets. In some cases, a normal (i.e. Gaussian) distribution of the offsets may be assumed if no additional data is available. In other cases, more complex probability distributions (e.g. different functions, including even asymmetric distributions) could be used. The probability distribution which is utilized may be computed based on theoretical assumptions, or could be based on an empirically acquired dataset of signal offsets, which is then statistically analyzed to identify a probability distribution.

The probability density function values at the calibration points can be used directly as weighting coefficients when calculating the calibration coefficients, or the weighting coefficients are derived as a function of the probability density function.

Where more than one output vector is applied in computing the output measurement(s), it may be advantageous to employ slightly different linearization functions (or at least differently fitted/optimized versions of the same linearization function) for different of the output vectors. For instance, depending upon the particular mapping operation which is applied by a given output vector, the resulting non-linearity in the output measurement error may differ. Therefore, having a linearization function which is specific to a given one or more of the different output vectors may improve error reduction in the output measurement(s).

Thus, according to some embodiments, the measurement operation may make reference to a dataset of multiple linearization functions (or multiple differently fitted/optimized versions of a same linearization function, i.e. having different sets of fitted parameters), wherein each linearization function is associated with only a subset of the plurality of output vectors, and wherein only the linearization function associated with a given output vector is applied to a given input channel in advance of applying the respective output vector.

Additionally or alternatively, in some examples, the measurement operation may employ a plurality of different linearization functions, for application to different subsets of one or more of the input channels. There may be a different respective linearization function for each input channel. This may allow for differences in the non-linear transfer functions along different input channel paths to be taken into account.

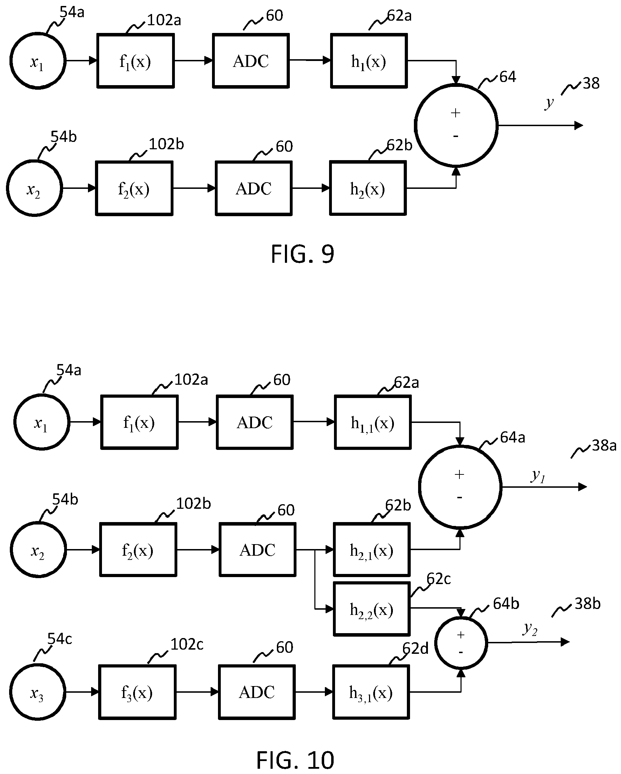

By way of illustration, Figs. 9 and 10 show two example processing flows for the measurement operation. In the example of Fig. 9, there are two input signals supplied to the measurement device: a first 54a input signal and a second 54b input signal. Each input signal is assumed to be subject to a respective non-linear transfer function 102a, 102b, which introduces an error to the signal which is a non-linear function of the input signal itself. Analog-digital conversion 60 is applied to the received signal at the measurement device. In the example of Fig. 9, each received input signal is processed with a different respective linearization function 62a, 62b. The two are then provided as inputs to a computation algorithm 64 which generates an output measurement 38 consisting of a linear combination of the corrected input signals.

The example of Fig. 10 is similar except that there are three input signals 54a, 54b, 54c, which are each assumed to be subject to a different respective non-linear transfer function 102a, 102b, 102c. In this example, two different output measurements 38a, 38b are obtained from application of two different computation algorithms 64a, 64b. The first computation algorithm receives only a subset of the input signals, in particular the first 54a and second 54b input signals (after linearization). The second computation algorithm 64b also receives only a subset of the input signals, in particular the second 54b and third 54c input signals (after linearization). Each computation algorithm 64a, 64b is associated with a different subset of a plurality of linearization functions 62a, 62, 62c, 62d. In particular, linearization functions 62a and 62b are associated with the first computation algorithm 64a, and wherein the first input signal 54a is processed with the first 62a linearization function and the second input signal 54b processed with the second linearization function 64b in advance of application of the first computation algorithm 64a. Linearization functions 62c and 62d are associated with the second computation algorithm 64b, and wherein the second input signal 54b is processed with the third linearization function 62c and the third input signal 54c processed with the fourth linearization function 64d in advance of application of the second computation algorithm 64b.

Above has been described the fact that the output measurement vectors may be taken into account when performing the calibration, e.g. to minimize an error between an expected output and actual output from application of a measurement vector to one or more input calibration signals.

The illustrative examples discussed so far have mentioned specifically only a system with two input channels. With only two input channels, only one vector is possible for computing the measurement, this corresponding to a computation of the difference between the two input channels.

By way of further illustrative example, a system might be considered having three input channels, which may be labelled x1, x2 and x3. In this case, three computation vectors are possible, for computing differences between each individual signal and each of the other two signals. However, computation vectors can also use "virtual" points, example x1 - (0.5*x2 - 0.5*x3), so that an infinite number of computation vectors is in fact theoretically possible. Which vectors are used will depend upon the application, i.e. which physiological signal is being measured. For example, in the context of a 12-lead ECG measurement device, twelve vectors may typically be computed, corresponding to difference signals between different combination of nine input electrodes (three limb electrodes RA, LA, LL, and six chest electrodes VI... V6). Other measurement modalities such as EEG may compute vectors between every pair of electrodes.

Thus, knowledge of the vectors used in the computation procedure can be used to reduce the set of calibration points that must be considered for each vector during the calibration operation. If two calibration points (where a calibration point means a particular set of input channel signals) differ only in terms of input channels that are utilized by a given vector, then only one of the points needs to be considered in the cost function for this vector.

For example, in an ECG with multiple chest electrodes (VI, V2, ...), no vectors are used that compute differences between two different chest electrodes.

The principles of the invention can be applied to any type of sensing or measurement device. Examples include electromedical measurement devices such as ECG measurement devices, electroanatomical imaging systems, EEG recorders, AED devices, fetal/obstetric ECG recorders, electroanatomical imaging systems. However, application is not limited to electromedical devices, and could be employed in other fields where differential measurements are acquired. For example, embodiments could be usefully applied in applications outside of clinical contexts, such as in smart watches or fitness trackers with ECG functionality. Embodiments of the invention might be usefully applied in any differential measurement device where common-mode to differential-mode conversion via input channel transfer function nonlinearities presents a problem.

By way of brief summary, the features of one particularly advantageous (although non-exhaustive) set of embodiments of the present invention might be summarized as follows.

An aim of at least one set of embodiments is to address the problem of common-mode to differential-mode conversion by input channel transfer function nonlinearities by providing a measurement device that applies linearization functions $h(x)$ to the input signals before calculating the difference or vector that is the output. The linearization functions use parameters which are determined in a calibration procedure by applying a sequence of constant input signals to the inputs of the device and recording the (raw/uncalibrated) output. The information in the set of input and output values, along with the information about the vectors used by the device and the statistical distribution of the offset values (or combinations of offset values) is then used to calculate linearization parameters that provide optimal linearization for likely combinations of channel offset values, and less optimal linearization for uncommon combinations. As the uncommon combinations usually indicate some problem with the measurement, like poor skin-electrode contact, this provides graceful degradation of the output measurements when the offset conditions change from good/common to poor/uncommon.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A computer-implemented method for implementation on a measurement device (22), comprising:

    a differential measurement operation, comprising:

        reading in signals (54a, 54b) from at least two input channels (32a, 32b) of the measurement device, the signal from each input channel being received from a respective sensor element (52a, 52b);
        applying a linearization function (62) to each of the input channels, the linearization function having adjustable parameters;
        applying a computation algorithm (64) to the input channels subsequent to applying the linearization function, to derive at least one output measurement (38);
        outputting a data signal (42) indicative of the output measurement; and

    a calibration operation performed at a different time than the measurement operation comprising:

        applying a plurality of sets (72) of constant-value calibration input signals (74a, 74b) to the input channels (32a, 32b) of the measurement device;
        applying the computation algorithm (64) to each set of the calibration input signals without applying the linearization function, and recording each respective output measurement, to form a calibration dataset (44); and
        fitting the adjustable parameters of the linearization function based on the calibration dataset.

2. A method as claimed in claim 1, wherein the linearization function is a polynomial function.

3. A method as claimed in claim 1 or 2, wherein the signal received at each input channel is assumed to represent a true sensing signal which has been processed by a non-linear transfer function, and wherein fitting the adjustable parameters of the linearization function comprises minimizing, for the calibration dataset, an error between a theoretical true output measurement for each calibration input signal, as would be obtained if the non-linear transfer function were not applied to the input signals, and the actual output measurement as recorded in the calibration dataset.

4. A method as claimed in any of claims 1-3, wherein the computation algorithm comprises application of one or more vectors to the input channels, each vector defining a mapping from the set of input channels to an output measurement, where the output measurement is a linear combination of the input channels with weightings defined by elements of the vector, and wherein the calibration is performed based in part on the one or more vectors.

5. A method as claimed in claim 4, and wherein the measurement operation comprises application of a plurality of said vectors for deriving a plurality of different output measurements.

6. A method as claimed in claim 5, wherein the measurement device comprises more than two input channels, wherein each of the plurality of vectors defines a mapping from only a subset of the input channels.

7. A method as claimed in claim 6, wherein the measurement operation comprises reference to a dataset of multiple linearization functions, wherein each linearization function is associated with only a subset of the plurality of vectors, and wherein only the linearization function associated with a given vector is applied to a given input channel in advance of applying the respective vector.

8. A method as claimed in any of claims 1-7, wherein the input channels are each assumed to comprise a differential mode component, a common-mode component, and an offset, and wherein the calibration procedure comprises reference to a predetermined probability distribution for the offsets of the input channels.

9. A method as claimed in claim 8, wherein the linearization function, and the calibration operation, is configured such that the calibrated linearization function provides more optimal linearization for a set of offsets which has a highest probability in the probability distribution, and less optimal linearization for a set of offsets which has lower probability in the probability distribution.

10. A method as claimed in claim 9,

   wherein the signal received at each input channel is assumed to represent a true sensing signal which has been processed by a non-linear transfer function, and wherein fitting the adjustable parameters of the linearization function comprises an optimization process in which an error between a true output measurement for a set of calibration input signals, as would be obtained if the non-linear transfer function were not applied to the input signals, and the actual output measurement as recorded in the calibration dataset, is minimized, and wherein more optimal linearization for a set of possible offsets means, after calibration, a smaller average error in the output measurements for input signals having said possible set of offsets, and a less optimal linearization for a set of possible offsets means, after calibration, a greater average error in the output measurements for input signals having said possible set of offsets.

11. A computer program product comprising code means configured, when run on a processor, to cause the processor to perform a method in accordance with any of claims 1-10.

12. A processing arrangement comprising:

   an input/output; and
   one or more processors,
   wherein the one or more processors are operable in a first mode in which the one or more processors perform a differential measurement operation, comprising:

      reading in signals from at least two input channels of the measurement device, the signal from each channel being received from a respective sensor element;
      applying a linearization function to each of the input channels, the linearization function having adjustable parameters;
      applying a computation algorithm to the input channels subsequent to applying the linearization function, to derive at least one output measurement;
      outputting a data signal indicative of the output measurement;

   and wherein the one or more processors are operable in a second mode in which they are configured to perform a calibration operation, comprising:

      applying a plurality of sets of constant-value calibration input signals to the input channels of the measurement device;
      applying the computation algorithm to each set of the calibration input signals without applying the linearization function, and recording each respective output measurement, to form a calibration dataset; and
      fitting the adjustable parameters of the linearization function based on the calibration dataset.

13. A measurement device comprising:

   at least two signal input ports for simultaneously reading in at least two input signal channels from respective sensor elements; and
a processing arrangement as claimed in claim 12.

14. A system, comprising:

a measurement device as claimed in claim 13, and
a signal measurement apparatus comprising at least two sensor elements for connection to the signal input ports.

FIG. 1

(a)

(b)

$$x1 = 0.5*d + c + \text{offset } 1$$
$$x2 = -0.5*d + c + \text{offset } 2$$

(c)

FIG. 2

52a

54a

I/O 26

Proc 24

32a

Input
Channel A

32b

Input
Channel B

42

Measurement
signal

36

Output

52b

22

38

Measurement

**FIG. 3**

52a

I/O 26

Proc

22

32a

Input
Channel A

62

Linearize

64

Computation

24

52b

34b

Input
Channel B

42

Measurement
signal

36

Output

38

Measurement

**FIG. 4**

FIG. 5

**Scaled probability density function (Channel 1 + Channel 2) / 2 = 0.5**

FIG. 6

FIG. 7

FIG. 8

FIG. 9

FIG. 10

<table>
<tr><td>Europäisches Patentamt<br>European Patent Office<br>Office européen des brevets</td><td>**EUROPEAN SEARCH REPORT**</td><td>Application Number<br>**EP 21 20 6863**</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2011/251817 A1 (BURNS MARK [US] ET AL) 13 October 2011 (2011-10-13) * figures 1, 3 * * paragraphs [0005], [0041], [0046], [0049] - [0053] * | 1-14 | INV.<br>A61B5/305<br>A61B5/00 |
| A | WO 85/04474 A1 (ROSEMOUNT INC [US]) 10 October 1985 (1985-10-10) * figure 1 * * pages 2-4, 6-8 * | 1-14 | |
| A | US 2014/368366 A1 (GALTON IAN [US] ET AL) 18 December 2014 (2014-12-18) * figure 5 * * paragraphs [0007], [0027], [0038] * | 1-14 | |
| A | KR 2019 0036959 A (KUMOH MACH & ELEC CO LTD [KR]; NOVASEN CO LTD [KR]) 5 April 2019 (2019-04-05) * paragraphs [0034] - [0035] * | 1-14 | TECHNICAL FIELDS SEARCHED (IPC)<br><br>A61B |

The present search report has been drawn up for all claims

| Place of search | Date of completion of the search | Examiner |
|---|---|---|
| The Hague | 10 May 2022 | Vanderperren, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 6863

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2022

| Patent document cited in search report | | Publication date | Patent family member(s) | | Publication date |
|---|---|---|---|---|---|
| US 2011251817 | A1 | 13-10-2011 | BR 112012026270 | A2 | 27-02-2018 |
| | | | EP 2557989 | A2 | 20-02-2013 |
| | | | JP 3183032 | U | 25-04-2013 |
| | | | US 2011251817 | A1 | 13-10-2011 |
| | | | WO 2011130291 | A2 | 20-10-2011 |
| WO 8504474 | A1 | 10-10-1985 | AU 570958 | B2 | 31-03-1988 |
| | | | EP 0177491 | A1 | 16-04-1986 |
| | | | JP S61501654 | A | 07-08-1986 |
| | | | WO 8504474 | A1 | 10-10-1985 |
| US 2014368366 | A1 | 18-12-2014 | NONE | | |
| KR 20190036959 | A | 05-04-2019 | NONE | | |

EPO FORM P0459

For more details about this annex : see Official Journal of the European Patent Office, No. 12/82